# EUROPEAN PATENT SPECIFICATION

(11) **EP 2 649 989 B1**
(45) Date of publication and mention of the grant of the patent: **18.10.2017**
(21) Application number: 12164044.5
(22) Date of filing: 13.04.2012
(51) Int. Cl.: A61K 9/14, A61K 31/405

(54) **Method for preparing a solid dispersion, solid dispersion obtained thereby and use thereof**
Verfahren zur Herstellung einer Feststoffdispersion, damit hergestellte Feststoffdispersion und Verwendung dafür
Procédé de préparation d'une dispersion solide, dispersion solide ainsi obtenue et son utilisation

(43) Date of publication of application: 16.10.2013
(73) Proprietor: King Saud University, 11421 Riyadh (SA)
(72) Inventor: Alam, Mohd Aftab, 11333 Riyadh (SA); Almohizea, Abdullah M., 11333 Riyadh (SA); Aljuffali, Ibrahim A., 11421 Riyadh (SA); Al-Jenoobi, Fahad Ibrahim, 11333 Riyadh (SA)
(74) Representative: Goddar, Heinz J.

(56) References cited:
- WO-A1-02/05820
- WO-A2-01/80822
- WO-A2-02/098388
- S.M. VASIM: "Effervescent mixture based solid dispersion a novel approach for solubility enhancement", RESEARCH JOURNAL OF PHARMACY AND TECHNOLOGY, vol. 4, no. 11, 30 November 2011 (2011-11-30), pages 1682-1686, XP9161756, ISSN: 0974-3618
- BHATT SHAILENDRA, TRIVEDI PRITI: "Development of domperidone: polyethylene glycol 6000 fast dissolving tablets from solid dispersions using effervescent method", JOURNAL OF CHEMICAL AND PHARMACEUTICAL RESEARCH, vol. 3, no. 6, 31 December 2011 (2011-12-31), pages 889-898, XP002681243, ISSN: 0975-7384
- VERRECK G ET AL: "The effect of pressurized carbon dioxide as a temporary plasticizer and foaming agent on the hot stage extrusion process and extrudate properties of solid dispersions of itraconazole with PVP-VA 64", EUROPEAN JOURNAL OF PHARMACEUTICAL SCIENCES, ELSEVIER, AMSTERDAM, NL, vol. 26, no. 3-4, 1 November 2005 (2005-11-01), pages 349-358, XP027803700, ISSN: 0928-0987 [retrieved on 2005-11-01]

## Description

The present invention relates to a method for preparing a solid dispersion, a solid dispersion obtained thereby, a pharmaceutical composition comprising the solid dispersion and the use of the solid dispersion.

Product development scientists often encounter difficulties in solving the problem of poorly water soluble drugs in development of pharmaceutical dosage forms. Poorly soluble drugs feature high crystallinity and therefore low solubility in water. Due to the low solubility, the absorption of some drugs is dissolution rate limited.

Consequently, different pharmaceutical strategies were developed to improve the desired properties (e.g. solubility and bioavailability) of poorly soluble drugs. These strategies comprise particle size reduction, complexation, lipid formulation, co-crystallization and solid dispersions.

Cyclodextrins and their derivatives have been used to enhance the solubility of poorly water soluble drugs. In this case, the drug forms a physical complex with cyclodextrin which features a higher solubility than the drug alone. The drug-cyclodextrin-complex also has improved bioavailability.

Among various approaches of solubility improvement, solid dispersions have been used to enhance the solubility of poorly water soluble drugs.

In a solid dispersion, the poorly soluble drug is dispersed in a solid matrix. It can exist in molecular, amorphous or microcrystalline form which provides a fast dissolution rate and/or solubility. Several techniques have been developed to prepare solid dispersions, including coprecipitation (US 5,985,326, US 6,350,786, US 20110245305), fusion, spray-drying (US 5,955,475, US 7,008,640) and hot-melt extrusion (US 7,081,255; US 20110245305). Solid dispersions prepared from different methods can differ in their properties, such as porosity, surface area, density, stability, hygroscopicity, solubility and bioavailability.

Solid dispersions can be prepared with water soluble as well as with water insoluble materials. Solid dispersions using water-soluble carriers can be achieved through the process of co-melting, quick cooling and pulverizing. This involves melting of a poorly water soluble drug with the solid water soluble carrier to form a liquid or semi-solid mass and subsequent hardening by cooling to low temperatures. The dispersion is then pulverized, sieved, mixed with relatively large amounts of excipients and encapsulated into hard gelatin capsules or compressed into tablets. The reported methods also disclose the use of water soluble carriers in combination with surfactant or wetting agents (US 5,281,420, US 8,025,899).

US 20110020455 discloses a solid dispersion containing a powdery porous carrier. The dispersion improves the solubility of hardly soluble active ingredients.

US 6,350,786 discloses a solid dispersion using water-insoluble ionic polymers.

US 6,548,555 describes the use of ionic polymers, including hydroxypropylmethyl cellulose acetate succinate for preparing solid dispersions for improving the solubility and bioavailability of drugs.

US 20110059175 discloses solubility enhanced solid dispersions of graft co-polymers (e.g. polyvinyl alcohol-polyethylene glycol graft copolymer) with poorly soluble drugs such as itraconazole.

US 5,225,197, US 4,127,645, US 6,489,346, US 6,589,507, US 6,780,882 and US 7,919,126 disclose effervescent couples comprising a basic component and an acidic component. The basic component liberates carbon dioxide when it reacts with the acidic component.

WO 01/80822 A2 relates to effervescent granulates, wherein effervescence assists disintegration and taste masking. According to the examples, granules are formed containing the effervescent couple. This prior art document discloses materials comprising pristine, unreactant effervescent couple to enable reaction when contacted with water after preparation.

WO 02/098388 A2 discloses an effervescent formulation comprising a drug and an effervescent couple prepared by mixing a drug with either acid or basic component and afterwards mixing the other of the basic or acidic compound of the effervescent couple with a mixture prepared before.

Research Journal of Pharmacy and Technology, Vol. 4, No. 11, pages 1682-1686, relates to an effervescent mixture based solid dispersion. The formulation is used for solubility enhancement.

It is an object of the present invention to provide a method for preparing a solid dispersion for use in pharmaceutical preparations, especially for poorly soluble drugs, which overcomes the drawbacks of the prior art. Particularly, a solid dispersion shall be provided improving the solubility and/or dissolution rate of drugs in an easy, efficient and cheap way.

This object is achieved by a method for preparing a solid dispersion, comprising the steps:
a.1) melting of a mixture of at least one drug, at least one carrier and at least one acid, at least one carbonate and/or at least one bicarbonate and optinally at least one surfactant under effervescence of CO₂;
   or
a.2) melting of at least one of the constituents of the mixture according to step a.1) and adding the remaining constituents in arbitrary order, wherein either the at least one acid or the at least one carbonate and/or the at least one carbonate has/have to be added last, wherein the solid dispersion is prepared by assistance of effervescence, i.e. when adding a carbonate and/or bicarbonate to the melted mixture of drug, carrier and acid, a reaction between acid and carbonate and/or bicarbonate occurs resulting in effervescence;
b) solidifying the mixture obtained in step a.1) or a.2), preferably by cooling and optionally drying;
c) comminuting the mixture obtained in step b).

According to the present invention, the term "solid dispersion" is to be understood to be a dispersion containing one or more drugs in an inert carrier material at solid state. The solid dispersion of the present invention is prepared by assistance of effervescence, i.e. when adding a carbonate and/or bicarbonate to the melted mixture of drug, carrier and acid, a reaction between acid and carbonate and/or bicarbonate occurs resulting in effervescence, i.e. the release of CO₂.

In a preferred embodiment, the drug has a water solubility of less than 5 mg/ml, preferably the drug features a water solubility of less than 1 mg/ml. In the present application, the term "poorly soluble drug" is to be understood to have a respective water solubility of less than 5 mg/ml.

The drug used for preparing the inventive solid dispersion is especially a poorly soluble drug referring to pharmaceutical active substances, their salts, polymers, solvates, esters or the like. The solubility can be measured in water, simulated gastric or intestinal fluid and is preferably measured using aqueous solutions having a pH of 1-7. These drugs are known to be classified under Biopharmaceutics Classification System (BCS) class II or class IV.

Particularly preferred, cooling in step b) of the inventive method is carried out as fast as possible by using respective means, for example a refrigerator, ice, liquid nitrogen etc. Furthermore, cooling to a temperature below room temperature can be particularly advantageous.

Preferably, the drug is selected from the group consisting of anesthetic agents, ACE inhibiting agents, antithrombotic agents, anti-allergic agents, antibacterial agents, antibiotic agents, anticoagulant agents, anticancer agents, antidiabetic agents, antihypertension agents, antifungal agents, antihypotensive agents, antiinflammatory agents, antimicotic agents, antimigraine agents, antiparkinson agents, antirheumatic agents, antithrombins, antiviral agents, beta blocking agents, bronchospasmolytic agents, calcium antagonists, cardiovascular agents, cardiac glycosidic agents, carotenoids, cephalosporins, contraceptive agents, cytostatic agents, diuretic agents, enkephalins, fibrinolytic agents, growth hormones, immunosupressants, insulins, interferons, lactation inhibiting agents, lipid-lowering agents, lymphokines, neurologic agents, prostacyclins, prostaglandins, psycho-pharmaceutical agents, protease inhibitors, magnetic, contraceptives, sedative agents, sex hormones, somatostatins, steroid hormonal agents, vaccines, vasodilating agents, and vitamins, preferably the drug is selected from the group consisting of albendazole, albendazole sulfoxide, alfaxalone, acetyldigoxin, acyclovir analogs, alprostadil, aminofostin, anipamil, antithrombin III, atenolol, azidothymidine, beclobrate, beclomethasone, bleomycin, benzocaine, beta carotene, beta endorphin, beta interferon, bezafibrate, binovum, biperiden, bromazepam, bromocriptine, bucindolol, buflomedil, bupivacaine, busulfan, cadralazine, camptothecin, canthaxanthin, captopril, carbamazepine, carboprost, cefalexin, cefalotin, cefamandole, cefazedone, cefluoroxime, cefmenoxime, cefoperazone, cefotaxime, cefoxitin, cefsulodin, ceftizoxime, chlorambucil, chromoglycinic acid, ciclonicate, ciglitazone, clonidine, cortexolone, corticosterone, cortisol, cortisone, cyclophosphamide, cyclosporin A and other cyclosporins, cytarabine, desocryptin, desogestrel, dexamethasone esters such as the acetate, dezocine, diazepam, diclofenac, dideoxyadenosine, dideoxyinosine, digitoxin, digoxin, dihydroergotamine, dihydroergotoxin, diltiazem, dopamine antagonists, doxorubicin, econazole, endralazine, enkephalin, enalapril, epoprostenol, estradiol, estramustine, etofibrate, etoposide, felbamate, fenbendazole, fenofibrate, flunarizin, flurbiprofen, 5-fluorouracil, flurazepam, fosfomycin, fosmidomycin, furosemide, gallopamil, gamma interferon, gentamicin, gepefrine, gliclazide, glipizide, griseofulvin, haptoglobulin, hepatitis B vaccine, hydralazine, hydrochlorothiazide, hydrocortisone, ibuprofen, ibuproxam, indinavir, indomethacin, ketoconazole, ketoprofen, ketotifen, ketotifen fumarate, K-Strophanthin, labetalol, lidocaine, lidoflazine, lisuride, lisuride hydrogen maleate, lorazepam, lovastatin, mefenamic acid, melphalan, memantine, mesulergin, metergoline, methotrexate, methyldigoxin, methylprednisolone, metronidazole, metisoprenol, metipranolol, metkephamide, metolazone, metoprolol, metoprolol tartrate, miconazole, miconazole nitrate, minoxidil, misonidazol, molsidomine, nadolol, nafiverine, nafazatrom, naproxen, natural insulins, nesapidil, nicardipine, nicorandil, nifedipine, niludipin, nimodipine, nitrazepam, nitrendipine, nitrocamptothecin, 9-nitrocamptothecin, oxazepam, oxprenolol, oxytetracycline, penicillins such as penicillin G benethamine, penecillin O, phenylbutazone, picotamide, pindolol, piposulfan, piretanide, piribedil, piroxicam, pirprofen, plasminogenic activator, prednisolone, prednisone, pregneninolone, procarbazine, procaterol, progesterone, proinsulin, propafenone, propentofylline, propofol, propranolol, rifapentine, simvastatin, semi-synthetic insulins, sobrerol, somatostatin and its derivatives, somatotropin, stilamin, sulfinpyrazone, suloctidil, suprofen, sulprostone, synthetic insulins, talinolol, taxol, taxotere, testosterone, testosterone propionate, testosterone undecanoate, tetracaine HI, tiaramide HCl, tolmetin, tranilast, triquilar, tromantadine HCl, urokinase, valium, verapamil, vidarabine, vidarabine phosphate sodium salt, vinblastine, vinburnin, vincamine, vincristine, vindesine, vinpocetine, vitamin A, vitamin E succinate, most preferably the drug is selected from the group consisting of indometacin, meloxicam, naproxen, diclofenac and piroxicam.

More preferably, the carrier has a higher water solubility than the drug.

It is preferred, that the carrier is selected from the group consisting of polyvinylpyrrolidone (PVP), Poloxamer 188, high molecular weight polyethylene glycol (PEG-4000 to PEG-10000), hydroxypropyl methyl cellulose (HPMC), nicotinamide, Poloxamer 407, polyoxyethylene 32 distearate, xylitol, polyvinyl pyrrolidone-vinylacetate copolymer, urea, citric acid, vinyl acetate copolymer, acrylic polymers, gelatin, dextrin, succinic acid, lactose, mannitol, sorbitol, sucrose, glucose, and dextrin, preferably the carrier is selected from the group consisting of mannitol, xylitol, sucrose, sorbitol, lactose, glucose, urea, PEG-6000, most preferably the carrier is mannitol.

Common acrylic polymers can be, for example, the acrylic polymers of the Eudragit series, such as Eudragit E100.

In one embodiment of the present invention, an organic acid may be used both as carrier and acid component, such a organic acid might be, for example, citric acid.

In a further preferred embodiment, the drug to carrier ratio is in a range from 1:1 to 1:20 w/w, preferably in a range from 1:2 to 1:15 w/w, most preferably is in a range from 1:4 to 1:10 w/w.

In a most preferred embodiment, the acid is an organic acid, preferably the acid is selected from the group consisting of citric acid, tartaric acid, adipic acid, fumaric acid, lactic acid, maleic acid, alginic acid, ascorbic acid, malic acid, succinic acid, hexamic acid, potassium bitartrate, citrate, sodium dihydrogen phosphate, sodium hydrogen phosphate, sodium acid sulfite and/or the corresponding anhydrides, most preferably the acid is citric acid.

In another preferred embodiment, the carbonate or the bicarbonate is selected from the group consisting of sodium carbonate, potassium carbonate, calcium carbonate, sodium bicarbonate, potassium bicarbonate, lithium bicarbonate, calcium bicarbonates, sodium glycine carbonate, sodium sesquicarbonate, magnesium carbonate, L-lysine carbonate, arginine carbonate, zinc carbonate, lithium carbonate, preferably the carbonate is sodium bicarbonate. A sesquicarbonate is to be understood as a double salt of bicarbonate and carbonate, such as Na₃H(CO₃)₂.

It is also preferred, that the surfactant is a non-ionic surfactant, an anionic surfactant or a cationic surfactant, preferably the surfactant is selected from the group consisting of polyoxyethylene fatty alcohol ethers, polyethylene glycols, sorbitan fatty acid esters, polyoxyethylene fatty acid esters, sorbitan esters, glycerol monostearate, cetyl alcohol, cetostearyl alcohol, stearyl alcohol, poloxamers, polaxamines, synthetic phospholipids, quaternary ammonium compounds, benzalkonium chloride, cetyltrimethylammonium bromide, lauryldimethylbenzyl-ammonium chloride, potassium laurate, triethanolamine stearate, sodium lauryl sulfate, alkyl polyoxyethylene sulfates, dioctyl sodium sulfosuccinate, negatively charged phospholipids (phosphatidyl glycerol, phosphatidylinositol, phosphatidylserine, phosphatidic acid and their salts), and negatively charged glyceryl esters.

It is further preferred, that the surfactant concentration in the solid dispersion is in a range from 0.1 to 25 percent by weight, preferably in a range from 0.5 to 15 percent by weight, most preferably in the range from 1 to 10 percent by weight.

The object is further achieved by a solid dispersion prepared by the inventive method.

The object is also achieved by a pharmaceutical composition comprising the inventive solid dispersion.

Finally, the object is achieved by the use of the inventive solid dispersion for enhancing the water solubility of drugs.

Surprisingly, it was found that the inventive solid dispersion provides the possibility to enhance the solubility of a drug in an easy, effective and cheap way, particularly by the reaction of an acid and a carbon dioxide liberating compound in the presence of a molten mixture of a drug and a carrier, during the preparation of the solid dispersion.

Preferably, the carrier has a good water solubility. Good water solubility in terms of the present invention means that the carrier exhibits a solubility in water, aqueous solutions, such as simulated gastric or intestinal fluids, or in aqueous buffers of pH 1-7 which allows sufficient administration and release of the drugs when used as or in a pharmaceutical composition.

Further, the carrier has to be inert and chemically compatible with the drug in molten as well as in solid state. To allow an efficient reaction of the acid and the carbon dioxide liberating compound an equinormal ratio of both should be chosen in step a.1) or a.2) of the inventive method. In this way, neutralisation of the acid as well as of the carbonate and/or bicarbonate compounds is achieved. If only partial neutralisation is accomplished, the end product has to be pharmaceutical acceptable.

Besides this, all other ingredients of the solid dispersion and of a pharmaceutical composition comprising the solid dispersion have to be pharmaceutically acceptable. Pharmaceutically acceptable in terms of the present invention means at least non-toxic.

Particularly advantageously, comminuting in step c) of the inventive method is micronising which means that the inventive solid dispersion will preferably show a grain size in a range from about 50 nm to several hundred µm.

It will be understood by a person skilled in the art that during step a.1) or a.2) of the inventive method mixing the components to achieve a substantially uniform dispersion can be ensured, for example by stirring. Further, it is clear that step a.1) or a.2) of the inventive method will be carried out until the end of the reaction of the acid and the carbon dioxide liberating compound, indicated by ceasing or significant decreasing of the effervescence.

The term bicarbonate as used in the present invention is synonymous to hydrogen carbonate.

The pharmaceutical composition of the present invention can be formulated in pharmaceutical dosage forms, such as solid, semisolid or liquid formulations for oral, rectal, vaginal, transdermal, sublingual, nasal, ocular or buccal administration.

It will also be appreciated that several of these dosage forms may include a range of excipients such as disintegrants, diluents, binders, lubricants, glidants, gelling agents, release modifier, surfactants/ solubilizers, emulsifiers, sweeteners, bioadhesive polymers, colourants and flavours.

Examples of suitable binders include hydroxypropyl cellulose, methyl cellulose, ethyl cellulose, sugars, povidone, gelatin, gum arabic, polyvinyl alcohol, pullulan, pregelatinized starch, agar, tragacanth, sodium alginate, and the like.

Examples of suitable diluents include microcrystalline cellulose, powdered cellulose, starch, starch pregelatinized, dextrates, lactitol, fructose, sugar compressible, sugar confectioners, dextrose, calcium phosphate-dibasic, calcium phosphate-tribasic, calcium sulfate, and the like.

The preferred diluents include microcrystalline cellulose, and starch.

Examples of suitable disintegrants include croscarmellose sodium, crospovidone and sodium starch glycolate, Low substituted-HPC and the like.

Examples of suitable sweeteners include mono-saccharide alcohols and disaccharide alcohols. The monosaccharide alcohols having 2-5 carbon atoms. They also include tetritols, pentitols, hexitols and heptitols. Examples of sugar alcohols include erythritol, mannitol, allitol, theritol, lactitol, ribitol, glucitol, arabinitol, dulcitol, altritol, iditol, maltitol, isomalt, hydrogenated starch hydrolysate and the like. Examples of non nutritive sweeteners include L-sugars, aspartame, alitame, acesulfame-K, cyclamate, stevioside, glycyrrhizin, sucralose, neohesperidin, dihydrochalcone, thaumatin saccharin and its pharmaceutically acceptable salts (e.g., calcium), and the like.

Examples of suitable release modifiers include cellulosic ether polymers (hydroxyethyl cellulose, hydroxypropyl cellulose and carboxy methylcellulose, ethyl cellulose) natural gums (guar gum, gum acacia, tragacanth, xantham, alginate), resins, other cellulose derivatives for enteric coating, polyacrylate copolymers, and the like.

Examples of suitable gelling agents include guar gum, gum acacia, tragacanth, xantham, alginate, hydroxypropyl cellulose and sodium carboxy methylcellulose, carbopol, and the like.

Examples of suitable bioadhesive polymers include, carbopol, natural gums, and the like.

Examples of suitable surfactants/ solubilizers include both non-ionic and ionic (cationic, anionic and zwitterionic) surfactants suitable for use in pharmaceutical dosage forms. These include polyethoxylated fatty acids and their derivatives, for example, polyethylene glycol 400 distearate, polyethylene glycol-20 dioleate, polyethylene glycol 4 150 mono dilaurate, polyethylene glycol-20 glyceryl stearate; alcohol-oil transesterification products, for example, polyethylene glycol-6 corn oil; polyglycerized fatty acids, for example, polyglyceryl-6 pentaoleate; propylene glycol fatty acid esters, for example, propylene glycol monocaprylate; mono and diglycerides, for example, glyceryl ricinoleate; sterol and sterol derivatives; sorbitan fatty acid esters and their derivatives, for example, polyethylene glycol-20 sorbitan monooleate, sorbitan monolaurate; polyethylene glycol alkyl ether or phenols, for example, polyethylene glycol-20 cetyl ether, polyethylene glycol-10-100 nonyl phenol; sugar esters, for example, sucrose monopalmitate; ionic surfactants, for example, sodium caproate, sodium glycocholate, soy lecithin, sodium stearyl fumarate, propylene glycol alginate, octyl sulfosuccinate disodium, palmitoyl carnitine; and the like.

Examples of suitable lubricants/glidants include magnesium stearate, magnesium silicate, hydrogenated vegetable oils, sodium stearyl fumarate, calcium stearate, colloidal silicon dioxide, aerosil, stearic acid, sodium lauryl sulphate, sodium benzoate, talc, hydrogenated castor oil, sucrose esters of fatty acid, microcrystalline wax, yellow beeswax, white beeswax, and the like.

Excipients such as colouring agents and pigments may also be added to dosage forms in accordance with the present invention, and suitable colouring agents and pigments may include titanium dioxide and dyes suitable for food. Preferred coloring agents include any FDA colors approved for oral use.

Flavours may be chosen from synthetic flavour oils and flavouring aromatics or natural oils, extracts from plants, leaves, flowers, fruits and so forth and combinations thereof. These may include cinnamon oil, oil of wintergreen, peppermint oils, bay oil, anise oil, eucalyptus, thyme oil. Also useful as flavours are vanilla, citrus oil, including lemon, orange, grape, lime and grapefruit, and fruit essences including apple, banana, pear, peach, strawberry, raspberry, cherry, plum, pineapple, apricot and so forth.

The invention will now be described in more detail by the following examples with the intention to exemplify the invention. The examples, however, are not intended to have a limiting effect on the subject-matter of the claims or on the scope of protection.

### EXAMPLES

### Example 1:

Mannitol (5.0 g) and citric acid (0.5 g) were melted by heating. The melting temperature was kept 10°C above the melting point of the material. Indomethacin (0.5 g) was added to the melted mixture of mannitol and citric acid, and was allowed to melt. Sodium bicarbonate (0.6 g) was added to the melted mixture of indomethacin, mannitol and citric acid under fast stirring. The melted effervescing mixture was stirred until effervescence ceased. The melted composition was cooled to low temperatures by means of a refrigerator. The cooled solid dispersion was dried, micronized and stored in desiccator.

### Example 2:

Mannitol (1.0 g) and citric acid (0.5 g) were melted by heating. The melting temperature was kept 10°C above the melting point of the material. Indomethacin (0.5 g) was added to the melted mixture of mannitol and citric acid and was allowed to melt. Sodium bicarbonate (0.6 g) was added to the melted mixture of indomethacin, mannitol and citric acid under fast stirring. The melted effervescing mixture was stirred until effervescence cease. The melted composition was cooled to low temperatures by means of a refrigerator. The cooled solid dispersion was dried, micronized and stored in desiccator.

### Example 3:

Citric acid (0.5 g) and indomethacin (0.5 g) were melted by heating. The melting temperature was kept 10°C above the melting point of the material. Sodium bicarbonate (0.6 g) was added to the melted mixture of indomethacin and citric acid under fast stirring. The melted effervescing mixture was stirred until effervescence ceased. The melted composition was cooled to low temperatures by means of a refrigerator. The cooled solid dispersion was dried, micronized and stored in desiccator.

### Example 4:

Mannitol (10.0 g) and citric acid (0.5 g) were melted by heating. The melting temperature was kept 10°C above the melting point of the material. Indomethacin (0.5 g) was added to the melted mixture of mannitol and citric acid and was allowed to melt. Sodium bicarbonate (0.6 g) was added to the melted mixture of indomethacin, mannitol and citric acid under fast stirring. The melted effervescing mixture was stirred until effervescence ceased. Melted composition was cooled to low temperatures by means of a refrigerator. The cooled solid dispersion was dried, micronized and stored in desiccator.

### Comparative Example 5:

Mannitol (10.0 g) was melted by heating. The melting temperature was kept 10°C above the melting point of the material. Indomethacin (0.5 g) was added to the melted mannitol and was allowed to melt. The melted composition was stirred and cooled to low temperatures by means of a refrigerator. The cooled solid dispersion was dried, micronized and stored in desiccator.

### Example 6:

Urea (5.0 g) and citric acid (0.5 g) were melted by heating. The melting temperature was kept 10°C above the melting point of the material. Indomethacin (0.5 g) was added to the melted mixture of urea and citric acid and was allowed to melt. Sodium bicarbonate (0.6 g) was added to the melted mixture of indomethacin, urea and citric acid under fast stirring. The melted effervescing mixture was stirred until effervescence ceased. The melted composition was cooled to low temperatures by means of a refrigerator. The cooled solid dispersion was dried, micronized and stored in desiccator.

### Comparative Example 7:

Urea (10g) was melted by heating. The melting temperature was kept 10°C above the melting point of the material. Indomethacin (0.5 g) was added to the melted urea, and was allowed to melt. The melted composition was stirred and cooled to low temperatures by means of a refrigerator. The cooled solid dispersion was dried, micronized and stored in desiccator.

### Example 8:

Mannitol (2.5 g) and citric acid (0.5 g) were melted by heating. The melting temperature was kept 10°C above the melting point of the material. Indomethacin (0.5 g) was added to the melted mixture of mannitol and citric acid and was allowed to melt. Sodium bicarbonate (0.6 g) was added to the melted mixture of indomethacin, mannitol and citric acid under fast stirring. The melted effervescing mixture was stirred until effervescence ceased. The melted composition was cooled to low temperatures by means of a refrigerator. The cooled solid dispersion was dried, micronized and stored in desiccator.

### Example 9:

The solubility of the drug (indomethacin) and solid dispersions of the drug according to the invention was examined in water according to the Higuchi-Connor method, see "Advances in Analytical Chemical Chemistry and Instrumentation" Vol. 4, New York, Wiley-Interscience 1965, page 117-212. An excess amount of drug, comparative solid dispersions and inventive solid dispersions was added to 10 ml of water, separately. The dispersions were shaken on mechanical shaker for 48 hours. Afterwards, the samples were withdrawn, filtered, diluted and analyzed to calculate the dissolved drug content.

Solubility results show that the solubility of indomethacin was significantly enhanced in effervescence assisted solid dispersions (EASD) as compared to the conventional solid dispersions (See Table 1).

**Table 1:**

| Example | Indomethacin Solubility in water (mg/ml) |
|---|---|
| Indomethacin | 0.152 |
| Example 1 | 3.56 |
| Example 2 | 0.786 |
| Example 3 | 0.841 |
| Example 4 | 0.364 |
| Comparative Example 5 | 0.277 |
| Example 6 | 6.11 |
| Comparative Example 7 | 0.212 |
| Example 8 | 4.27 |

### Example 10:

The effervescent assisted solid dispersion of indomethacin (I ndomethacin+ mannitol + citric acid + sodium bicarbonate) of the invention was uniformly mixed with microcrystalline cellulose & crosspovidone and the mixture was lubricated with magnesium stearate. This lubricated blend was filled into hard gelatin capsules by tapping procedure. The composition of capsule is given under table 2.

**Table 2:**

| Excipients | Qty (mg) | Qty 50 Units (mg) |
|---|---|---|
| Indomethacin | 25 | 1250 |
| Organic acid | 25 | 1250 |
| NaHCO₃ | 30 | 1500 |
| Crosspovidone | 20 | 1000 |
| MCC | 35 | 1750 |
| Mannitol | 125 | 6250 |
| Mg-stearate | 1.5 | 75 |

| | | |
|---|---|---|
| **Qty = Quantity** | | |

### Example 11:

The USP paddle method was used for in-vitro release studies. Dissolution was carried out in 900 ml water at 37 ± .05 °C. Paddle speed was set at 50 rpm. Samples (5 ml) were withdrawn at different time intervals and replaced with fresh dissolution media. The samples were analyzed by using UV-spectrophotometer. The dissolution of marked capsule and capsule filled with effervescence assisted solid dispersion was compared. More than 80% w/w of indomethacin was released in 10 minutes from capsules comprising effervescent assisted solid dispersion of the invention. The brand capsule releases about only 70% of indomethacin.

The features disclosed in the foregoing description and in the claims may, both separately and in any combination thereof, be material for realizing the invention in diverse forms thereof.

## Claims

1. Method for preparing a solid dispersion, comprising the steps:
a.1) melting of a mixture of at least one drug, at least one carrier and at least one acid, at least one carbonate and/or at least one bicarbonate and optionally at least one surfactant under effervescence of CO₂;
or
a.2) melting of at least one of the constituents of the mixture according to step a.1) and adding the remaining constituents in arbitrary order, wherein either the at least one acid or the at least one bicarbonate and/or the at least one carbonate has/have to be added last, wherein the solid dispersion is prepared by assistance of effervescence, i.e. when adding a carbonate and/or bicarbonate to the melted mixture of drug, carrier and acid, a reaction between acid and carbonate and/or bicarbonate occurs resulting in effervescence;
b) solidifying the mixture obtained in step a.1) or a.2), preferably by cooling and optionally drying;
c) comminuting the mixture obtained in step b).

2. Method according to claim 1, wherein the drug has a water solubility of less than 5 mg/ml, preferably less than 1 mg/ml.

3. Method according to claim 1 or 2, wherein the at least one drug is selected from the group consisting of anesthetic agents, ACE inhibiting agents, antithrombotic agents, anti-allergic agents, antibacterial agents, antibiotic agents, anticoagulant agents, anticancer agents, antidiabetic agents, antihypertension agents, antifungal agents, antihypotensive agents, antiinflammatory agents, antimicotic agents, antimigraine agents, antiparkinson agents, antirheumatic agents, antithrombins, antiviral agents, beta blocking agents, bronchospasmolytic agents, calcium antagonists, cardiovascular agents, cardiac glycosidic agents, carotenoids, cephalosporins, contraceptive agents, cytostatic agents, diuretic agents, enkephalins, fibrinolytic agents, growth hormones, immunosupressants, insulins, interferons, lactation inhibiting agents, lipid-lowering agents, lymphokines, neurologic agents, prostacyclins, prostaglandins, psycho-pharmaceutical agents, protease inhibitors, magnetic, contraceptives, sedative agents, sex hormones, somatostatins, steroid hormonal agents, vaccines, vasodilating agents, and vitamins, preferably the drug is selected from the group consisting of albendazole, albendazole sulfoxide, alfaxalone, acetyldigoxin, acyclovir analogs, alprostadil, aminofostin, anipamil, antithrombin III, atenolol, azidothymidine, beclobrate, beclomethasone, bleomycin, benzocaine, beta carotene, beta endorphin, beta interferon, bezafibrate, binovum, biperiden, bromazepam, bromocriptine, bucindolol, buflomedil, bupivacaine, busulfan, cadralazine, camptothecin, canthaxanthin, captopril, carbamazepine, carboprost, cefalexin, cefalotin, cefamandole, cefazedone, cefluoroxime, cefmenoxime, cefoperazone, cefotaxime, cefoxitin, cefsulodin, ceftizoxime, chlorambucil, chromoglycinic acid, ciclonicate, ciglitazone, clonidine, cortexolone, corticosterone, cortisol, cortisone, cyclophosphamide, cyclosporin A and other cyclosporins, cytarabine, desocryptin, desogestrel, dexamethasone esters such as the acetate, dezocine, diazepam, diclofenac, dideoxyadenosine, dideoxyinosine, digitoxin, digoxin, dihydroergotamine, dihydroergotoxin, diltiazem, dopamine antagonists, doxorubicin, econazole, endralazine, enkephalin, enalapril, epoprostenol, estradiol, estramustine, etofibrate, etoposide, felbamate, fenbendazole, fenofibrate, flunarizin, flurbiprofen, 5-fluorouracil, flurazepam, fosfomycin, fosmidomycin, furosemide, gallopamil, gamma interferon, gentamicin, gepefrine, gliclazide, glipizide, griseofulvin, haptoglobulin, hepatitis B vaccine, hydralazine, hydrochlorothiazide, hydrocortisone, ibuprofen, ibuproxam, indinavir, indomethacin, ketoconazole, ketoprofen, ketotifen, ketotifen fumarate, K-Strophanthin, labetalol, lidocaine, lidoflazine, lisuride, lisuride hydrogen maleate, lorazepam, lovastatin, mefenamic acid, melphalan, memantine, mesulergin, metergoline, methotrexate, methyldigoxin, methylprednisolone, metronidazole, metisoprenol, metipranolol, metkephamide, metolazone, metoprolol, metoprolol tartrate, miconazole, miconazole nitrate, minoxidil, misonidazol, molsidomine, nadolol, nafiverine, nafazatrom, naproxen, natural insulins, nesapidil, nicardipine, nicorandil, nifedipine, niludipin, nimodipine, nitrazepam, nitrendipine, nitrocamptothecin, 9-nitrocamptothecin, oxazepam, oxprenolol, oxytetracycline, penicillins such as penicillin G benethamine, penicillin O, phenylbutazone, picotamide, pindolol, piposulfan, piretanide, piribedil, piroxicam, pirprofen, plasminogenic activator, prednisolone, prednisone, pregneninolone, procarbazine, procaterol, progesterone, proinsulin, propafenone, propentofylline, propofol, propranolol, rifapentine, simvastatin, semi-synthetic insulins, sobrerol, somatostatin and its derivatives, somatotropin, stilamin, sulfinpyrazone, suloctidil, suprofen, sulprostone, synthetic insulins, talinolol, taxol, taxotere, testosterone, testosterone propionate, testosterone undecanoate, tetracaine HI, tiaramide HCl, tolmetin, tranilast, triquilar, tromantadine HCl, urokinase, valium, verapamil, vidarabine, vidarabine phosphate sodium salt, vinblastine, vinburnin, vincamine, vincristine, vindesine, vinpocetine, vitamin A, vitamin E succinate, most preferably the drug is selected from the group consisting of indometacin, meloxicam, naproxen, diclofenac and piroxicam.

4. Method according to any of the preceding claims, wherein the carrier has a higher water solubility than the drug.

5. Method according to any of the preceding claims, wherein the carrier is selected from the group consisting of polyvinylpyrrolidone (PVP), Poloxamer 188, high molecular weight polyethylene glycol (PEG-4000 to PEG-10000), hydroxypropyl methyl cellulose (HPMC), nicotinamide, poloxamer 407, Polyoxyethylene 32 distearate, xylitol, polyvinyl pyrrolidone-vinylacetate copolymer, urea, citric acid, vinyl acetate copolymer, acrylic polymers, gelatin, dextrin, succinic acid, lactose, mannitol, sorbitol, sucrose, glucose, and dextrin, preferably the carrier is selected from the group consisting of mannitol, xylitol, sucrose, sorbitol, lactose, glucose, urea, PEG-6000, most preferably the carrier is mannitol.

6. Method according to any of the preceding claims, wherein the drug to carrier ratio is in a range from 1:1 to 1:20 w/w, preferably in a range from 1:2 to 1:15 w/w, most preferably in a range from 1:4 to 1:10 w/w.

7. Method according to any of the preceding claims, wherein the acid is an organic acid, preferably the acid is selected from the group consisting of citric acid, tartaric acid, adipic acid, fumaric acid, lactic acid, maleic acid, alginic acid, ascorbic acid, malic acid, succinic acid, hexamic acid, potassium bitartrate, citrate, sodium dihydrogen phosphate, sodium hydrogen phosphate, sodium acid sulfite and/or the corresponding anhydrides, most preferably the acid is citric acid.

8. Method according to any of the preceding claims, wherein the carbonate or the bicarbonate is selected from the group consisting of sodium carbonate, potassium carbonate, calcium carbonate, sodium bicarbonate, potassium bicarbonate, lithium bicarbonate, calcium bicarbonates, sodium glycine carbonate, sodium sesquicarbonate, magnesium carbonate, L-lysine carbonate, arginine carbonate, zinc carbonate, lithium carbonate, preferably the bicarbonate is sodium bicarbonate.

9. Method according to any of the preceding claims, wherein the surfactant is a non-ionic surfactant, an anionic surfactant or a cationic surfactant, preferably the surfactant is selected from the group consisting of polyoxyethylene fatty alcohol ethers, polyethylene glycols, sorbitan fatty acid esters, polyoxyethylene fatty acid esters, sorbitan esters, glycerol monostearate, cetyl alcohol, cetostearyl alcohol, stearyl alcohol, poloxamers, polaxamines, synthetic phospholipids, quaternary ammonium compounds, benzalkonium chloride, cetyltrimethylammonium bromide, lauryldimethylbenzyl-ammonium chloride, potassium laurate, triethanolamine stearate, sodium lauryl sulfate, alkyl polyoxyethylene sulfates, dioctyl sodium sulfosuccinate, negatively charged phospholipids (phosphatidyl glycerol, phosphatidylinositol, phosphatidylserine, phosphatidic acid and their salts), and negatively charged glyceryl esters.

10. Method according to any of the preceding claims, wherein the surfactant concentration in the solid dispersion is in a range from 0.1 to 25 percent by weight, preferably in a range from 0.5 to 15 percent by weight, most preferably in a range from 1 to 10 percent by weight.

11. Solid dispersion prepared by a method according to any of the claims 1 to 10.

12. Pharmaceutical composition comprising the solid dispersion according to claim 11.

13. Use of the solid dispersion according to claim 10 for enhancing the water solubility of drugs.

## Patentansprüche

1. Verfahren zum Herstellen einer festen Dispersion, umfassend die Schritte:
a.1) Schmelzen eines Gemisches zumindest eines Medikaments, zumindest eines Trägers und zumindest einer Säure, zumindest eines Carbonats und/oder zumindest eines Bicarbonats und optional zumindest einer oberflächenaktiven Substanz unter Schaumbildung durch CO₂;
oder
a.2) Schmelzen von zumindest einem der Bestandteile des Gemisches nach Schritt a.1) und Hinzufügen der übrigen Bestandteile in beliebiger Reihenfolge, wobei entweder die zumindest eine Säure oder das zumindest eine Bicarbonat und/oder das zumindest eine Carbonat zuletzt hinzugegeben wird, wobei die feste Dispersion unter Einfluss von Sprudeln hergestellt wird, d.h. durch die Zugabe eines Carbonats und/oder Bicarbonats zu dem geschmolzenen Gemisch eines Medikaments, Trägers und Säure, findet eine Reaktion zwischen der Säure und dem Carbonat und/oder Bicarbonat statt, was zum Sprudeln führt;
b) Verfestigen des Gemischs, das in Schritt a.1) oder a.2) erhalten wird, vorzugsweise durch Kühlen und optionales Trocknen;
c) Zerkleinern des Gemisches, das in Schritt b) erhalten wird.

2. Verfahren nach Anspruch 1, wobei das Medikament eine Wasserlöslichkeit von weniger als 5 mg/ml, vorzugsweise weniger als 1 mg/ml, hat.

3. Verfahren nach Anspruch 1 oder 2, wobei das zumindest eine Medikament ausgewählt ist aus der Gruppe, bestehend aus Anästhesiemitteln, ACE-Inhibitoren, Anti-Gerinnungsmitteln, Antiallergika, antibakteriellen Mitteln, Antibiotika, Anti-Gerinnungshemmern, Antikrebsmedikamenten, Antidiabetika, blutdrucksenkenden Mitteln, Antimykotika, blutdrucksenkenden Mitteln, entzündungshemmenden Mitteln, antimykotischen Mitteln, Antimigränemitteln, Antiparkinsonmitteln, antirheumatischen Mitteln, Antithrombinen, antiviralen Mitteln, Betablockern, bronchospasmolytischen Mitteln, Calciumantagonisten, kardiovaskulären Mitteln, Herz-Glykoside-Mitteln, Carotenoiden, Cephalosporine, Verhütungsmitteln, zytostatischen Mitteln, Diuretika, Enkephalinen, fibrinolytischen Mitteln, Wachstumshormonen, Immunosupressiva, Insulinen, Interferonen, laktationshemmende Mittel, Lipid-senkende Mittel, Lymphokinen, neurologischen Mitteln, Prostacyclinen, Prostaglandinen, Psychopharmaceuticas, Proteaseinhibitoren, magnetische, Verhütungsmitteln, Beruhigungsmitteln, Sexualhormone, Somatostatinen, Steroid-Hormonmittel, Impfstoffe, vasodilatierenden Mitteln und Vitaminen, vorzugsweise ist das Arzneinmittel ausgewält aus der Gruppe bestehend aus Albendazol, Albendazolsulfoxid, Alfaxalon, Acetyldigoxin, Acyclovir-Analoga, Alprostadil, Aminofostin, Anipamil, Antithrombin III, Atenolol, Azidothymidin, Beclobrat, Beclomethason, Bleomycin, Benzocain, Beta-Carotin, Beta-Eendorphin, Beta-Interferon, Bezafibrat, Binovum, Biperiden, Bromazepam, Bromocriptin, Bucindolol, Buflomedil, Bupivacaine, Busulfan, Cadralazin, Camptothecin, Canthaxanthin, Captopril, Carbamazepin, Carboprost, Cefalexin, Cefalotin, Cefamandol, Cefazedon, Cefluoroxim, Cefmenoxim, Cefoperazon, Cefotaxim, Cefoxitin, Cefsulodin, Ceftizoxim, Chlorambucil, Chromoglycinsäure, Ciclonicate, Ciglitazon, Clonidin, Cortexolon, Corticosteron, Cortisol, Cortison, Cyclophosphamid, Cyclosporin A und andere Cyclosporine, Cytarabin, Desocryptin, Desogestrel, Dexamethasonester wie Acetat, Dezocin, Diazepam, Diclofenac, Dideoxyadenosin, Dideoxyinosin, Digitoxin, Digoxin, Dihydroergotamin, Dihydroergotoxin, Diltiazem, Dopaminantagonisten, Doxorubicin, Econazol, Endralazin, Enkephalin, Enalapril, Epoprostenol, Estradiol, Estramustin, Etofibrat, Etoposid, Felbamat, Fenbendazol, Fenofibrat, flunarizin, Flurbiprofen, 5-Fluorouracil, Flurazepam, Fosfomycin, Fosmidomycin, Furosemid, Gallopamil, gamma-Interferon, Gentamicin, Gepefrin, Gliclazid, Glipizid, Griseofulvin, Aptoglobulin, Hepatitis B-Impfstoff, Hydralazin, Hydrochlorothiazid, Hydrocortison, Ibuprofen, Ibuproxam, Indinavir, Indomethacin, Ketoconazol, Ketoprofen, Ketotifen, Ketotifen Fumarat, K-Strophanthin, Labetalol, Lidocain, Lidoflazin, Lisurid, Lisurid Wasserstoffmaleat, Lorazepam, Lovastatin, Mefenamicsäure, Melphalan, Memantin, Mesulergin, Metergolin, Methotrexat, Methyldigoxin, Methylprednisolon, Metronidazol, Metisoprenol, Metipranolol, Metkephamid, Metolazon, Metoprolol, Wasserstoffmaleat, Miconazol, Miconazol Nitrat, Minoxidil, Misonidazol, Molsidomin, Nadolol, Nafiverin, Nafazatrom, Naproxen, natürlichen Insulinen, Nesapidil, Nicardipin, Nicorandil, Nifedipin, Niludipin, Nimodipin, Nitrazepam, Nitrendipin, Nitrocamptothecin, 9-Nitrocamptothecin, Oxazepam, Oxprenolol, Oxytetracyclin, Penicillinen wie Penicillin G-Benethamin, Penicillin O, Phenylbutazon, Picotamid, Pindolol, Piposulfan, Piretanid, Piribedil, Piroxicam, Pirprofen, Plasminogenaktivator, Prednisolon, Prednison, Pregneninolon, Procarbazin, Procaterol, Progesteron, Proinsulin, Propafenon, Propentofyllin, Propofol, Propranolol, Rifapentin, Simvastatin, halbsynthetischen Insulinen, Sobrerol, Somatostatin und seine Derivative, Somatotropin, Stilamin, Sulfinpyrazon, Suloctidil, Suprofen, Sulproston, synthetischen Insulinen, Talinolol, Taxol, Taxoter, Testosteron, Testosteronpropionat, Testosteronundecanoat, Tetracain HCl, Tiaramid HCl, Tolmetin, Tranilast, Triquilar, tromantadin HCl, Urokinas, Valium, Verapamil, Vidarabin, Vidarabinphosphatnatriumsalz, Vinblastin, Vinburnin, Vincamin, Vincristin, Vindesin, Vinpocetin, Vitamin A, Vitamin E-Succinat, bevorzugter Arzneimittel ausgewählt aus der Gruppe bestehend aus Indometacin, Meloxicam, Naproxen, Diclofenac und Piroxicam.

4. Verfahren nach einem der vorangehenden Ansprüche, wobei der Träger eine höhere Wasserlöslichkeit als das Medikament hat.

5. Verfahren nach einem der vorangehenden Ansprüche, wobei der Träger ausgewählt ist aus der Gruppe, bestehend aus Polyvinylpyrrolidon (PVP), Poloxamer 188, Polyethylenglykol eines hohen Molekulargewichts (PEG-4000 bis PEG-10000), Hydroxypropylmethylcellulose (HPMC), Nikotinamid, Poloxamer 407, Polyoxyethylen-32-distearat, Xylitol, Polyvinylpyrrolidonvinylacetat-Copolymer, Harnstoff, Citronensäure, Vinylacetat-Copolymer, akrylischen Polymeren, Gelatine, Dextrin, Bernsteinsäure, Lactose, Mannitol, Sorbitol, Saccharose, Glucose und Dextrin, vorzugsweise der Träger ausgewählt ist aus der Gruppe, bestehend aus Mannitol, Xylitol, Saccharose, Sorbitol, Lactose, Glucose, Harnstoff, PEG-6000, am meisten bevorzugt Mannitol.

6. Verfahren nach einem der vorangehenden Ansprüche, wobei das Medikamentzu-Träger-Verhältnis in einem Bereich von 1:1 bis 1:20 Gew./Gew., vorzugsweise in einem Bereich von 1:2 bis 1:15 Gew./Gew., am meisten bevorzugt in einem Bereich von 1:4 bis 1:10 Gew./Gew. ist.

7. Verfahren nach einem der vorangehenden Ansprüche, wobei die Säure eine organische Säure ist, vorzugsweise die Säure ausgewählt ist aus der Gruppe, bestehend aus Citronensäure, Weinsäure, Adipinsäure, Fumarsäure, Milchsäure, Maleinsäure, Alginsäure, Ascorbinsäure, Malonsäure, Bernsteinsäure, Hexansäure, Kaliumbitartrat, Citrat, Natriumdihydrogenphosphat, Natriumhydrogenphosphat, Natriumsäuresulfit und/oder den korrespondierenden Anhydriden, vorzugsweise die Säure Citronensäure ist.

8. Verfahren nach einem der vorangehenden Ansprüche, wobei das Carbonat oder Bicarbonat ausgewählt ist aus der Gruppe, bestehend aus Natriumcarbonat, Kaliumcarbonat, Calciumcarbonat, Natriumbicarbonat, Kaliumbicarbonat, Lithiumbicarbonat, Calciumbicarbonaten, Natriumglycincarbonat, Natriumsesquicarbonat, Magnesiumcarbonat, L-Lysincarbonat, Arginincarbonat, Zinkcarbonat, Lithiumcarbonat, vorzugsweise ist das Bicarbonat Natriumbicarbonat.

9. Verfahren nach einem der vorangehenden Ansprüche, wobei das oberflächenaktive Mittel ein nicht-ionisches oberflächenaktives Mittel, ein anionisches oberflächenaktives Mittel, eine kationisches oberflächenaktives Mittel ist, vorzugsweise das oberflächenaktive Mittel ausgewählt ist aus der Gruppe, bestehend aus Polyoxyethylen-Fettalkohol-Ether, Polyoxyethylen-Fettalkohol-Ethern, Polyethylenglykolen, Sorbitanfettsäureestern, Polyoxyethylenfettsäureestern, Sorbitanestern, Glycerolmonostearat, Cetylalcohol, Cetostearylalcohol, Stearylalcohol, Poloxameren, Polaxaminen, synthetische Phospholipiden, quaternären Ammoniumverbindungen, Benzalkoniumchlorid, Cetyltrimethylammoniumbromid, Lauryldimethylbenzylammoniumchlorid, Kaliumlaurat, Triethanolaminstearat, Natriumlaurylsulfat, Alkylpolyoxyethylensulfaten, Dioctylnatriumsulfosuccinaten, negativ belasteten Phospholipiden (Phosphatidylglycerin, Phosphatidylinositol, Phosphatidylserin, Phosphatidsäure und deren Salzen) und negativ belasteten Glycerylestern.

10. Verfahren nach einem der vorangehenden Ansprüche, wobei die Konzentration des oberflächenaktiven Mittels in der festen Dispersion in einem Bereich von 0,1 bis 25 Gew.-%, vorzugsweise in einem Bereich von 0,5 bis 15 Gew.-%, am meisten bevorzugt in einem Bereich von 1 bis 10 Gew.-% ist.

11. Feste Dispersion, hergestellt durch ein Verfahren nach einem der Ansprüche 1 bis 10.

12. Pharmazeutische Zusammensetzung, umfassend die feste Dispersion nach Anspruch 11.

13. Verwendung der festen Dispersion nach Anspruch 10 zum Verbessern der Wasserlöslichkeit von Medikamenten.

## Revendications

1. Procédé de préparation d'une dispersion solide comprenant les étapes consistant à
a.1) faire fondre un mélange d'au moins un médicament, d'au moins un véhicule et d'au moins un acide, d'au moins un carbonate et/ou d'au moins un bicarbonate et, éventuellement, d'au moins un agent tensio-actif sous effervescence du CO₂ ;
ou
a.2) faire fondre au moins un des constituants du mélange selon l'étape a.1) et ajouter les constituants restants dans un ordre arbitraire, où soit le au moins un acide ou le au moins un bicarbonate et/ou le au moins un carbonate doi(ven)t être ajouté(s) en dernier et où la dispersion solide est préparée à l'aide d'une effervescence, c.-à-d. où l'addition d'un carbonate et/ou d'un bicarbonate au mélange fondu du médicament, du véhicule et de l'acide entraîne une réaction entre l'acide et le carbonate et/ou le bicarbonate qui résulte en une effervescence ;
b) solidifier le mélange obtenu à l'étape a.1) ou a.2), préférablement par refroidissement et éventuellement séchage ;
c) réduire en poudre le mélange obtenu à l'étape b).

2. Procédé selon la revendication 1, où le médicament a une solubilité dans l'eau inférieure à 5 mg/ml, préférablement inférieure à 1 mg/ml.

3. Procédé selon la revendication 1 ou 2, où le au moins un médicament est sélectionné dans le groupe consistant en des agents anesthésiques, agents inhibiteurs de l'ECA, agents antithrombotiques, agents antiallergiques, agents antibactériens, agents antibiotiques, agents anticoagulants, agents anticancéreux, agents antidiabétiques, agents antihypertensifs, agents antifongiques, agents antihypotensifs, agents anti-inflammatoires, agents antimicotiques, agents antimigraineux, agents antiparkinsoniens, agents antirhumatismaux, agents antithrombines, agents antiviraux, agents bêta-bloquants, agents bronchospsamolytiques, antagonistes calciques, agents cardio-vasculaires, agents glycosidiques cardiaques, caroténoïdes, céphalosporines, agents contraceptifs, agents cytostatiques, agents diurétiques, encéphalines, agents fibrinolytiques, hormones de croissance, immunosuppresseurs, insulines, interférons, agents inhibiteurs de la lactation, agents hypolipémiants, lymphokines, agents neurologiques, prostacyclines, prostaglandines, agents psycho-pharmaceutiques, inhibiteurs de protéase, agents magnétiques, contraceptifs, agents sédatifs, hormones sexuelles, somatostatines, agents hormonaux stéroïdiens, vaccins, agents vasodilatateurs et vitaminés, le médicament étant préférablement sélectionné dans le groupe consistant en les suivants : albendazole, sulfoxyde d'albendazole, alfaxalone, acétyldigoxine, analogues de l'acyclovir, alprostadil, amifostine, anipamil, antithrombine III, aténolol, azidothymidine, béclobrate, béclomethasone, bléomycine, benzocaïne, bêta-carotène, bêta-endorphine, interféron bêta, bézafibrate, binovum, bipéridène, bromazépam, bromocriptine, bucindolol, buflomédil, bupivacaïne, busulfan, cadralazine, camptothécine, canthaxanthine, captopril, carbamazépine, carboprost, céfalexine, céfalotine, céfamandole, céfazédone, céfluoroxime, cefménoxime, céfopérazone, céfotaxime, céfoxitine, cefsulodine, ceftizoxime, chlorambucil, acide chromoglycinique, ciclonicate, ciglitazone, clonidine, cortexolone, corticostérone, cortisol, cortisone, cyclophosphamide, cyclosporine A et autres cyclosporines, cytarabine, désocryptine, désogestrel, esters de dexaméthasone comme l'acétate, dézocine, diazépam, diclofénac, didésoxyadénosine, didésoxyinosine, digitoxine, digoxine, dihydroergotamine, dihydroergotoxine, diltiazem, antagonistes de la dopamine, doxorubicine, éconazole, endralazine, encéphaline, énalapril, époprosténol, eestradiol, estramustine, étofibrate, étoposide, felbamate, fenbendazole, fénofibrate, flunarizine, flurbiprofène, fluoro-5 uracile, flurazépam, fosfomycine, fosmidomycine, furosémide, gallopamil, interféron gamma, gentamicine, gépéfrine, gliclazide, glipizide, griséofulvine, haptoglobuline, vaccin contre l'hépatite B, hydralazine, hydrochlorothiazide, hydrocortisone, ibuprofène, ibuproxam, indinavir, indométacine, kétoconazole, kétoprofène, kétotifène, fumarate de kétotifène, strophanthine K, labétalol, lidocaïne, lidoflazine, lisuride, hydrogénomaléate de lisuride, lorazépam, lovastatine, acide méfénamique, melphalan, mémantine, mésulergine, métergoline, méthotrexate, méthyldigoxine, méthylprednisolone, métronidazole, métisoprinol, métipranolol, metképhamide, métolazone, métoprolol, tartrate de métoprolol, miconazole, nitrate de miconazole, minoxidil, misonidazole, molsidomine,, nadolol, nafivérine, nafazatrom, naproxène, insulines naturelles, nesapidil, nicardipine, nicorandil, nifédipine, niludipine, nimodipine, nitrazépam, nitrendipine, nitrocamptothécine, 9-nitrocamptothécine, oxazépam, oxprénolol, oxytétracycline, pénicillines comme la pénicilline G-bénéthamine, pénicilline O, phénylbutazone, picotamide, pindolol, piposulfan, pirétanide, piribédil, piroxicam, pirprofène, activateur du plasminogène, prednisolone, prednisone, prégnéninolone, procarbazine, procatérol, progestérone, proinsuline, propafénone, propentofylline, propofol, propranolol, rifapentine, simvastatine, insulines semi-synthétiques, sobrérol, somatostatine et ses dérivés, somatotropine, stilamine, sulfinpyrazone, suloctidil, suprofène, sulprostone, insulines synthétiques, talinolol, taxol, taxotere, testostérone, propionate de testostérone, undécanoate de testostérone, tétracaïne HCl, tiaramide HCl, tolmétine, tranilast, triquilar, tromantadine HCl, urokinase, valium, vérapamil, vidarabine, sel de sodium du phosphate de vidarabine, vinblastine, vinburnine, vincamine, vincristine, vindésine, vinpocétine, vitamine A et succinate de vitamine E, le médicament étant le plus préférablement sélectionné dans le groupe consistant en l'indométacine, le méloxicam, le naproxène, le diclofénac et le piroxicam.

4. Procédé selon l'une quelconque des revendications précédentes, où la solubilité dans l'eau du véhicule est plus élevée que celle du médicament.

5. Procédé selon l'une quelconque des revendications précédentes, où le véhiculé est sélectionné dans le groupe consistant en les suivants : poly(vinylpyrrolidone) (PVP), poloxamère 188, polyéthylène glycol de haut poids moléculaire (PEG-4 000 à PEG-10000), hydroxypropylméthylcellulose (HPMC), nicotinamide, poloxamère 407, distéarate de polyoxyéthylène 32, xylitol, copolymère de poly(vinylpyrrolidone) et d'acétate de vinyle, urée, acide citrique, copolymère d'acétate de vinyle, polymères acryliques, gélatine, dextrine, acide succinique, lactose, mannitol, sorbitol, sucrose, glucose et dextrine, le véhicule étant préférablement sélectionné dans le groupe consistant en le mannitol, le xylitol, le sucrose, le sorbitol, le lactose, le glucose, l'urée et le PEG-6 000 et étant le plus préférablement le mannitol.

6. Procédé selon l'une quelconque des revendications précédentes, où le rapport médicament/véhicule est dans une plage de 1/1 à 1/20 p/p, préférablement dans une plage de 1/2 à 1/15 p/p et le plus préférablement dans une plage de 1/4 à 1/10 p/p.

7. Procédé selon l'une quelconque des revendications précédentes où l'acide est un acide organique, l'acide étant préférablement sélectionné dans le groupe consistant en les suivants : acide citrique, acide tartrique, acide adipique, acide fumarique, acide lactique, acide maléique, acide alginique, acide ascorbique, acide malique, acide succinique, acide hexamique, bitartrate de potassium, citrate, dihydrogénophosphate de sodium, hydrogénophosphate de sodium, sulfite acide de sodium et/ou anhydrides correspondants et l'acide étant le plus préférablement l'acide citrique.

8. Procédé selon l'une quelconque des revendications précédentes, où le carbonate ou le bicarbonate est sélectionné dans le groupe consistant en les suivants : carbonate de sodium, carbonate de potassium, carbonate de calcium, bicarbonate de sodium, bicarbonate de potassium, bicarbonate de lithium, bicarbonates de calcium, carbonate de glycine sodique, sesquicarbonate de sodium, carbonate de magnésium, carbonate de L-lysine, carbonate d'arginine, carbonate de zinc et carbonate de lithium, le bicarbonate étant préférablement le bicarbonate de sodium.

9. Procédé selon l'une quelconque des revendications précédentes où l'agent tensio-actif est un agent tensio-actif non ionique, un agent tensio-actif anionique ou un agent tensio-actif cationique, l'agent tensio-actif étant préférablement sélectionné dans le groupe consistant en les suivants : éthers d'alcool gras et de polyoxyéthylène, polyéthylène glycols, esters d'acide gras et de sorbitane, esters d'acide gras et de polyoxyéthylène, esters de sorbitane, monostéarate de glycérol, alcool cétylique, alcool cétostéarylique, alcool stéarylique, poloxamères, polaxamines, phospholipides synthétiques, composés d'ammonium quaternaire, chlorure de benzalkonium, bromure de cétyltriméthylammonium, chlorure de lauryldiméthylbenzylammonium, laurate de potassium, stéarate de triéthanolamine, laurylsulfate de sodium, alkylsulfates de polyoxyéthylène, dioctylsulfosuccinate de sodium, phospholipides chargés négativement (phosphatidylglycérol, phosphatidylinositol, phosphatidylsérine, acide phosphatidique et leurs sels) et esters de glycéryle chargés négativement.

10. Procédé selon l'une quelconque des revendications précédentes, où la concentration en agent tensio-actif présente dans la dispersion solide est dans une plage de 0,1 à 25 pour cent en poids, préférablement dans une plage de 0,5 à 15 pour cent en poids, le plus préférablement dans une plage de 1 à 10 pour cent en poids.

11. Dispersion solide préparée par un procédé selon l'une quelconque des revendications 1 à 10.

12. Composition pharmaceutique comprenant la dispersion solide selon la revendication 11.

13. Utilisation de la dispersion solide selon la revendication 10 pour augmenter la solubilité dans l'eau de médicaments.
